# EUROPÄISCHE PATENTSCHRIFT

(11) **EP 3 404 622 B1**
(45) Veröffentlichungstag und Bekanntmachung des Hinweises auf die Patenterteilung: **26.06.2024**
(21) Anmeldenummer: 17172037.8
(22) Anmeldetag: 19.05.2017
(51) Int. Cl.: G06T 15/08, G06T 3/00

(54) **VERARBEITUNG VON MEDIZINISCHEN 3D-BILDDATEN EINES RIPPENKNOCHENS**
PROCESSING OF MEDICAL 3D IMAGE DATA OF A RIB BONE
TRAITEMENT DE DONNÉES D'IMAGE MÉDICALE 3D D'UNE CÔTE

(43) Veröffentlichungstag der Anmeldung: 21.11.2018
(73) Patentinhaber: Siemens Healthineers AG, 91301 Forchheim (DE)
(72) Erfinder: SOZA, Grzegorz, 90562 Heroldsberg (DE); GROSSKOPF, Stefan, 90408 Nürnberg (DE); MARTINKE, Hannes, 91325 Adelsdorf (DE); PETRY, Christian, 91094 Langensendelbach (DE); RINGL, Helmut, 2320 Schwechat (AT); SÜHLING, Michael, 91052 Erlangen (DE)
(74) Vertreter: Siemens Healthineers Patent Attorneys

(56) Entgegenhaltungen:
- WO-A1-2012/037091
- WO-A2-2006/000925
- US-A1- 2010 142 788
- US-A1- 2013 007 099
- US-A1- 2013 070 996
- US-A1- 2013 272 594
- Hannes Martink: "Advanced Bone Visualization Bachelor - Thesis in Study Computer Visualistics", , 13. März 2017 (2017-03-13), Seiten FP-V,1-71, XP002774431, Magdeburg Gefunden im Internet: URL:http://wwwisg.cs.uni-magdeburg.de/visu alisierung/wiki/data/media/files/misc/mart inke2017.pdf [gefunden am 2017-10-05] -& Anonymous: "Advanced Bone Visualization Bachelor - Thesis in Study Computer Visualistics - Google search", Google , 5. Oktober 2007 (2007-10-05), XP002774432, Gefunden im Internet: URL:https://www.google.nl/search?dcr=0&q=r ib+unfolding+filetype:pdf&oq=rib+unfolding +filetype:pdf&gs_l=psy-ab.3...23610326.236 13455.0.23614208.13.13.0.0.0.0.157.1658.0j 12.12.0....0...1.1.64.psy-ab..1.7.1026...3 3i160k1j33i21k1.0.Ubii-kkX4-4 [gefunden am 2017-10-05]
- HELMUT RINGL ET AL: "The ribs unfolded - a CT visualization algorithm for fast detection of rib fractures: effect on sensitivity and specificity in trauma patients", EUROPEAN RADIOLOGY, Bd. 25, Nr. 7, 14. Februar 2015 (2015-02-14), Seiten 1865-1874, XP055225320, DE ISSN: 0938-7994, DOI: 10.1007/s00330-015-3598-2
- SHIN B S ET AL: "Efficient unfolding of virtual endoscopy using linear ray interpolation", COMPUTER METHODS AND PROGRAMS IN BIOMEDICINE, ELSEVIER, AMSTERDAM, NL, vol. 93, no. 2, 1 February 2009 (2009-02-01), pages 174-184, XP025770037, ISSN: 0169-2607, DOI: 10.1016/J.CMPB.2008.09.003 [retrieved on 2008-10-23]
- Mistelbauer G. ET AL: "Vessel Visualization using Curvicircular Feature Aggregation", Computer graphics forum : journal of the European Association for Computer Graphics, vol. 32, no. 3pt2, 1 June 2013 (2013-06-01), pages 231-240, XP055804725, Oxford ISSN: 0167-7055, DOI: 10.1111/cgf.12110
- Kanitsar Armin ET AL: "Diagnostic Relevant Visualization of Vascular Structures" In: "Scientific Visualization: The Visual Extraction of Knowledge from Data", 1 January 2006 (2006-01-01), Springer Berlin Heidelberg, Berlin Heidelberg, XP055805544, ISBN: 978-3-540-26066-0 pages 207-228, DOI: 10.1007/3-540-30790-7_13, Retrieved from the Internet: URL:https://citeseerx.ist.psu.edu/viewdoc/ download?doi=10.1.1.147.3356&rep=rep1&type =pdf>

## Beschreibung

Die Erfindung betrifft ein Verfahren zur Bilddatenverarbeitung, eine Bilddatenverarbeitungseinheit und eine medizinische Bildgebungsvorrichtung.

Nach der Durchführung einer medizinischen Bildgebungsuntersuchung, beispielsweise eines CT-Scans, werden die Bilddaten in der Regel durch den befundenden Radiologen an einem Befundungsplatz beurteilt. Die Diagnose der Rippen anhand von CT-Bilddaten ist dem befundenen Radiologen mit bisherigen Methoden oft nur mit relativ hohem Aufwand möglich. Zur Diagnose von beispielsweise Rippenbrüchen kann der Radiologe entweder durch die am Scanner erstellten Schichtdaten navigieren oder eine spezielle Softwarelösung verwenden, welche die Rippen mithilfe der aus den Bilddaten ermittelten Rippen-Mittellinien in eine planare Ebene entfaltet. Insbesondere kann die Befundung der gekrümmten Rippen auf herkömmlichen planaren CT-Bildern, beispielsweise anhand von 300 bis 400 Bildern pro Patient, sehr mühsam sein.

Es gibt Visualisierungssoftware, welche es ermöglicht, die in planare Ebenen entfaltete Rippen zu beurteilen. Das Entfalten der Rippen in eine planare Ebene ist dem Fachmann insbesondere unter dem Begriff "Rib Unfolding" bekannt. Eine bekannte Softwarelösung hierfür ist syngo.via CT Bone Reading. Diese entfalteten Rippen bilden einen 3D-Bilddatensatz und werden typischerweise entlang von planaren Schnittebenen visualisiert, so dass in der Regel weitere Interaktionsschritte zur Befundung nötig sind.

US 9020233 B2 offenbart ein Verfahren zum Extrahieren einer Mittellinie einer Rippe in einem medizinischen 3D-Bilddatensatz.

US 9547906 B2 offenbart ein Verfahren zum Korrigieren einer Mittellinie einer Rippe, welche aus einem medizinischen 3D-Bilddatensatz extrahiert wurde.

US 9558568 B2 offenbart ein Verfahren zur Visualisierung eines Knochengerüsts mit einer Vielzahl von Knochen.

WO 2012/037091 A1 offenbart eine Methode zur Entfaltung volumetrischer Daten.

Helmut Ringl et al.: "The ribs unfolded - a CT visualization algorithm for fast detection of rib fractures: effect on sensitivity and specificity in trauma patients", EUROPEAN RADIOLOGY, Bd. 25, Nr. 7, 14. Februar 2015 (2015-02-14), Seiten 1865-1874, betrifft einen CT-Visualisierungsalgorithmus zur schnellen Erkennung von Rippenfrakturen.

WO 2006/000925 A2 offenbart ein virtuelles Endoskopieverfahren zur Durchführung einer virtuellen Endoskopie an einem volumetrischen Bild einer lumenbestimmenden Struktur mit einem länglichen Lumen.

Shin B.S. et al.: "Efficient unfolding of virtual endoscopy using linear ray interpolation", Computer Methods and Programs in Biomedicine, Elsevier, Bd. 93, Nr. 2, Februar 2009 (2009-02), Seiten 174-184, betrifft eine Entfaltung in der virtuellen Endoskopie durch lineare Strahleninterpolation.

US 2010/142788 A1 offenbart ein medizinisches Bildverarbeitungsgerät zur Visualisierung eines in Volumendaten enthaltenen röhrenförmigen Gewebes.

Mistelbauer G. et al.: "Vessel Visualization using Curvicircular Feature Aggregation", Computer Graphics Forum, Wiley-Blackwell, Bd. 32, Nr. 3, Juni 2013 (2013-06), Seiten 231-240, betrifft eine Visualisierung von Gefäßen durch kurvenförmige Merkmalsaggregation.

US 2013/272594 A1 offenbart ein Verfahren zur Bildgebung und/oder Diagnose eines knocheninneren Raumes.

Kanitsar A. et al.: "Diagnostic Relevant Visualization of Vascular Structures" In: "Scientific Visualization: The Visual Extraction of Knowledge from Data", 2006, Springer, Seiten 207-228, betrifft eine Visualisierung von vaskulären Strukturen.

Beim herkömmlichen Rib Unfolding ist es daher erforderlich, Visualisierungsparameter interaktiv anzupassen und durch 3D-Schichtbilder zu navigieren, da auch bei der entfalteten Ansicht in der Regel nur ein einziger Schnitt durch die Rippen sichtbar ist.

Die Erfindung hat die Aufgabe, eine verbesserte 2D-Darstellung von Rippen in einem medizinischen Bild zu ermöglichen.

Jeder der Gegenstände der unabhängigen Ansprüche löst jeweils diese Aufgabe. In den abhängigen Ansprüchen sind weitere vorteilhafte Aspekte der Erfindung berücksichtigt.

Die Erfindung betrifft ein von einem Computer ausgeführtes Verfahren zur Bilddatenverarbeitung, wobei das Verfahren die folgenden Schritte umfasst:
- Bereitstellen eines medizinischen 3D-Bilddatensatzes, welcher eine längliche anatomische Struktur betrifft, wobei in dem medizinischen 3D-Bilddatensatz eine Mittellinie der länglichen anatomischen Struktur definiert ist, wobei die längliche anatomische Struktur eine Rippe ist,
- Definieren zumindest einer gekrümmten Schicht in dem medizinischen 3D-Bilddatensatz, wobei sich die zumindest eine gekrümmte Schicht um die Mittellinie der länglichen anatomischen Struktur windet,
- Abtasten zumindest eines Teils des medizinischen 3D-Bilddatensatzes in die zumindest eine gekrümmte Schicht basierend auf der Mehrzahl von Strahlen,
- Abrollen der zumindest einen gekrümmten Schicht, in welche der zumindest eine Teil des medizinischen 3D-Bilddatensatzes abgetastet wurde, wobei zumindest eine abgerollte planare Schicht ermittelt wird.

Erfindungsgemäß handelt es sich bei der zumindest einen gekrümmten Schicht um eine Mehrzahl von gekrümmten Schichten. Erfindungsgemäß sind die gekrümmten Schichten der Mehrzahl von gekrümmten Schichten im Wesentlichen koaxial um die Mittellinie angeordnet.

Erfindungsgemäß ummanteln die gekrümmten Schichten der Mehrzahl von gekrümmten Schichten die Mittellinie der länglichen anatomischen Struktur jeweils schlauchförmig.

Erfindungsgemäß repräsentieren die gekrümmten Schichten der Mehrzahl von gekrümmten Schichten jeweils eine Fläche, welche durch eine zentrische Streckung auf die Oberfläche der länglichen anatomischen Struktur abgebildet werden kann. Insbesondere kann eine Mehrzahl von Strahlen definiert werden, welche jeweils radial in Bezug auf die Mittellinie der länglichen anatomischen Struktur verlaufen und welche die zumindest eine gekrümmte Schicht schneiden.

Erfindungsgemäß handelt es sich bei der zumindest einen abgerollten planaren Schicht um eine Mehrzahl von abgerollten planaren Schichten, welche zusammen einen Volumendatensatz bilden. Insbesondere kann der Volumendatensatz den zumindest einen Teil des medizinischen 3D-Bilddatensatzes repräsentieren.

Erfindungsgemäß wird basierend auf dem Volumendatensatz ein 2D-Bild mittels einer Minimum-Intensitäts-Projektion oder Maximum-Intensitäts-Projektion erzeugt.

Gemäß einer Ausführungsform der Erfindung ist vorgesehen, dass jeder Strahl der Mehrzahl von Strahlen von der Mittellinie der länglichen anatomischen Struktur weg gerichtet ist oder dass jeder Strahl der Mehrzahl von Strahlen auf die Mittellinie der länglichen anatomischen Struktur gerichtet ist. Gemäß einer Ausführungsform der Erfindung erfolgt das Abtasten des medizinischen 3D-Bilddatensatzes in die zumindest eine gekrümmte Schicht basierend auf der Mehrzahl von Strahlen unter Verwendung von Raytracing und/oder Raycasting.

Gemäß einer Ausführungsform der Erfindung wird eine Zuordnung von Punkten des medizinischen 3D-Bilddatensatzes zu Punkten der zumindest einen abgerollten planaren Schicht oder des 2D-Bildes ermittelt.

Gemäß einer Ausführungsform der Erfindung ist vorgesehen, dass es sich bei dem zumindest einen Teil des 3D-Bilddatensatzes um den 3D-Bilddatensatz handelt und/oder dass es sich bei dem zumindest einen Teil des 3D-Bilddatensatzes um diejenige Menge von Voxeln des 3D-Bilddatensatzes handelt, welche eine segmentierte Teilstruktur der länglichen anatomischen Struktur repräsentiert.

Damit ist beispielsweise eine eineindeutige Zuordnung zwischen Voxeln des 3D-Bilddatensatzes und Pixeln der abgerollten planaren Schicht realisierbar. Auf diese Weise kann vermieden werden, dass Features, die im 3D-Bilddatensatz vorhanden sind, beim Befunden anhand der abgerollten planaren Schicht übersehen werden.

Gemäß einer Ausführungsform der Erfindung wird zumindest eine Teilstruktur der länglichen anatomischen Struktur segmentiert. Insbesondere kann der zumindest eine Teil des medizinischen 3D-Bilddatensatzes, welcher in die zumindest eine gekrümmte Schicht abgetastet wird, basierend auf der zumindest einen Teilstruktur ermittelt werden. Insbesondere kann zunächst die Mittellinie der länglichen anatomischen Struktur ermittelt werden. Die längliche anatomische Struktur kann beispielsweise ausgehend von der ermittelten Mittellinie der länglichen anatomischen Struktur segmentiert werden. Die längliche anatomische Struktur kann beispielsweise unter Verwendung einer bekannten Methode zur Segmentierung, beispielsweise einer Region-Growing-basierten Methode oder einer Oval-Methode, segmentiert werden.

Bei der Rippe kann es sich insbesondere um eine entfaltete Rippe handeln. Die Rippe kann beispielsweise unter Verwendung einer bekannten Methode zur Rippen-Entfaltung entfaltet werden.

Die Erfindung betrifft ferner eine Bilddatenverarbeitungseinheit, aufweisend:
- eine Bereitstellungseinheit, welche ausgebildet ist zum Bereitstellen eines medizinischen 3D-Bilddatensatzes, welcher eine längliche anatomische Struktur betrifft, wobei in dem medizinischen 3D-Bilddatensatz eine Mittellinie der länglichen anatomischen Struktur definiert ist, wobei die längliche anatomische Struktur eine Rippe ist,
- eine Schicht-Definitionseinheit, welche ausgebildet ist zum Definieren zumindest einer gekrümmten Schicht in dem medizinischen 3D-Bilddatensatz, wobei sich die zumindest eine gekrümmte Schicht um die Mittellinie der länglichen anatomischen Struktur windet,
- eine Strahlen-Definitionseinheit, welche ausgebildet ist zum Definieren einer Mehrzahl von Strahlen, welche jeweils radial in Bezug auf die Mittellinie der länglichen anatomischen Struktur verlaufen und die zumindest eine gekrümmte Schicht schneiden,
- eine Abtasteinheit, welche ausgebildet ist zum Abtasten zumindest eines Teils des medizinischen 3D-Bilddatensatzes in die zumindest eine gekrümmte Schicht basierend auf der Mehrzahl von Strahlen,
- eine Abrolleinheit, welche ausgebildet ist zum Abrollen der zumindest einen gekrümmten Schicht, in welche der zumindest eine Teil des medizinischen 3D-Bilddatensatzes abgetastet wurde, wobei zumindest eine abgerollte planare Schicht ermittelt wird,
- wobei die Bilddatenverarbeitungseinheit dazu eingerichtet ist, ein erfindungsgemäßes Verfahren auszuführen.

Gemäß einer Ausführungsform der Erfindung weist die Bilddatenverarbeitungseinheit eine Strahlen-Definitionseinheit auf, welche ausgebildet ist zum Definieren einer Mehrzahl von Strahlen, welche jeweils radial in Bezug auf die Mittellinie der länglichen anatomischen Struktur verlaufen und die zumindest eine gekrümmte Schicht schneiden.

Die Erfindung betrifft ferner eine medizinische Bildgebungsvorrichtung, aufweisend die Bilddatenverarbeitungseinheit.

Die Erfindung betrifft ferner ein Computerprogrammprodukt mit einem Computerprogramm, welches direkt in eine Speichereinrichtung einer Bilddatenverarbeitungseinheit einer medizinischen Bildgebungsvorrichtung ladbar ist, mit Programmabschnitten, um alle Schritte eines erfindungsgemäßen Verfahrens auszuführen, wenn das Computerprogramm in der Bilddatenverarbeitungseinheit der medizinischen Bildgebungsvorrichtung ausgeführt wird.

Die Erfindung betrifft ferner ein computerlesbares Medium, auf welchem von einem Computer einlesbare und ausführbare Programmabschnitte gespeichert sind, um alle Schritte eines erfindungsgemäßen Verfahrens auszuführen, wenn die Programmabschnitte von dem Computer ausgeführt werden.

Die medizinische Bildgebungsvorrichtung kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus, insbesondere einem PET-CT-Gerät und einem PET-MR-Gerät, besteht. Die medizinische Bildgebungsvorrichtung kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen.

Ohne Einschränkung des allgemeinen Erfindungsgedankens wird bei einigen der Ausführungsformen ein Computertomographiegerät beispielhaft für eine medizinische Bildgebungsvorrichtung genannt.

Die Bilddatenverarbeitungseinheit und/oder eine oder mehrere Komponenten der Bilddatenverarbeitungseinheit können von einem Datenverarbeitungssystem gebildet sein. Das Datenverarbeitungssystem kann beispielsweise eine oder mehrere Komponenten in Form von Hardware und/oder eine oder mehrere Komponenten in Form von Software aufweisen. Das Datenverarbeitungssystem kann beispielsweise zumindest teilweise von einem Cloud-Computing-System gebildet sein.

Das Datenverarbeitungssystem kann beispielsweise ein Cloud-Computing-System, ein Computernetzwerk, ein Computer, ein Tabletcomputer, ein Smartphone oder ähnliches oder Kombinationen davon sein und/oder aufweisen.

Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Field-programmable gate array), ein ASIC-System (Application-specific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen.

Insbesondere kann eine Komponente der Bilddatenverarbeitungseinheit nach einem der Aspekte, die in dieser Anmeldung offenbart sind, welche dazu ausgebildet ist, einen gegebenen Schritt eines Verfahrens nach einem der Aspekte, die in dieser Anmeldung offenbart sind, auszuführen, in Form einer Hardware implementiert sein, welche zum Ausführen des gegebenen Schritts konfiguriert ist und/oder welche zum Ausführen einer computerlesbaren Anweisung derart konfiguriert ist, dass die Hardware mittels der computerlesbaren Anweisung zum Ausführen des gegebenen Schritts konfigurierbar ist. Insbesondere kann das System einen Speicherbereich, beispielsweise in Form eines computerlesbaren Mediums, aufweisen, in welchem computerlesbare Anweisungen, beispielsweise in Form eines Computerprogramms, gespeichert sind.

Ein Datentransfer zwischen Komponenten des Datenverarbeitungssystems kann beispielsweise jeweils mittels einer geeigneten Datentransfer-Schnittstelle erfolgen. Die Datentransfer-Schnittstelle zum Datentransfer an und/oder von einer Komponente des Datenverarbeitungssystems kann zumindest teilweise in Form von Software und/oder zumindest teilweise in Form von Hardware realisiert sein. Die Datentransfer-Schnittstelle kann beispielsweise zum Abspeichern von Daten in und/oder zum Laden von Daten aus einem Bereich des Speichersystems ausgebildet sein, wobei auf diesen Bereich des Speichersystems eine oder mehrere Komponenten des Datenverarbeitungssystems zugreifen können.

Insbesondere können Daten, welche beispielsweise ein medizinisches Bild betreffen, insbesondere der 3D-Bilddatensatz, bereitgestellt werden, indem die Daten geladen, beispielsweise aus einem Bereich eines Speichersystems geladen, und/oder erzeugt, beispielsweise mittels einer medizinischen Bildgebungsvorrichtung erzeugt, werden.

Das Computerprogramm ist in das Speichersystem des Datenverarbeitungssystems ladbar und von dem Prozessorsystem des Datenverarbeitungssystems ausführbar. Das Datenverarbeitungssystem kann beispielsweise mittels des Computerprogramms derart ausgebildet sein, dass das Datenverarbeitungssystem die Schritte eines Verfahrens nach einer der Ausführungsformen, die in dieser Anmeldung offenbart sind, ausführen kann, wenn das Computerprogramm von dem Datenverarbeitungssystem ausgeführt wird.

Das Computerprogrammprodukt kann beispielsweise das Computerprogramm sein oder neben dem Computerprogramm mindestens einen zusätzlichen Bestandteil umfassen. Der mindestens eine zusätzliche Bestandteil des Computerprogrammprodukts kann als Hardware und/oder als Software ausgebildet sein.

Das Computerprogrammprodukt kann beispielsweise ein Speichermedium, auf dem zumindest ein Teil des Computerprogrammprodukts gespeichert ist, und/oder ein Schlüssel zur Authentifizierung eines Benutzers des Computerprogrammprodukts, insbesondere in Form eines Dongles, aufweisen. Das Computerprogrammprodukt und/oder das Computerprogrammkann beispielsweise ein Cloud-Anwendungs-Programm aufweisen, welches zum Verteilen von Programmabschnitten des Computerprogramms auf verschiedene Verarbeitungseinheiten, insbesondere verschiedene Computer, eines Cloud-Computing-Systems ausgebildet ist, wobei jede der Verarbeitungseinheiten zum Ausführen eines oder mehrerer Programmabschnitte des Computerprogramms ausgebildet ist.

Auf dem computerlesbaren Medium kann beispielsweise das Computerprogrammprodukt nach einer der Ausführungsformen, die in dieser Anmeldung offenbart sind, und/oder das Computerprogramm nach einer der Ausführungsformen, die in dieser Anmeldung offenbart sind, gespeichert sein. Das computerlesbare Medium kann beispielsweise ein Memorystick, eine Festplatte oder ein sonstiger Datenträger sein, der insbesondere lösbar mit dem Datenverarbeitungssystem verbunden oder fest in das Datenverarbeitungssystem integriert sein kann. Das computerlesbare Medium kann beispielsweise einen Bereich des Speichersystems des Datenverarbeitungssystems bilden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
Fig. 1 ein Blockschema eines Ausführungsbeispiels eines erfindungsgemäßen Verfahrens zur Bilddatenverarbeitung,
Fig. 2 eine schematische Ansicht eines Ausführungsbeispiels einer erfindungsgemäßen Bilddatenverarbeitungseinheit,
Fig. 3 ein medizinisches Bild mit entfalteten Rippen,
Fig. 4 eine schematische Ansicht, außerhalb der Erfindung, eines Querschnitts eines Beispiels für eine längliche anatomische Struktur und zumindest eine gekrümmte Schicht,
Fig. 5 eine schematische Ansicht eines Querschnitts eines weiteren Beispiels für eine längliche anatomische Struktur und zumindest eine gekrümmte Schicht,
Fig. 6 eine schematische Darstellung des Abrollens von gekrümmten Schichten in planare Schichten,
Fig. 7 eine schematische Ansicht, außerhalb der Erfindung, eines Querschnitts eines weiteren Beispiels für eine längliche anatomische Struktur und zumindest eine gekrümmte Schicht,

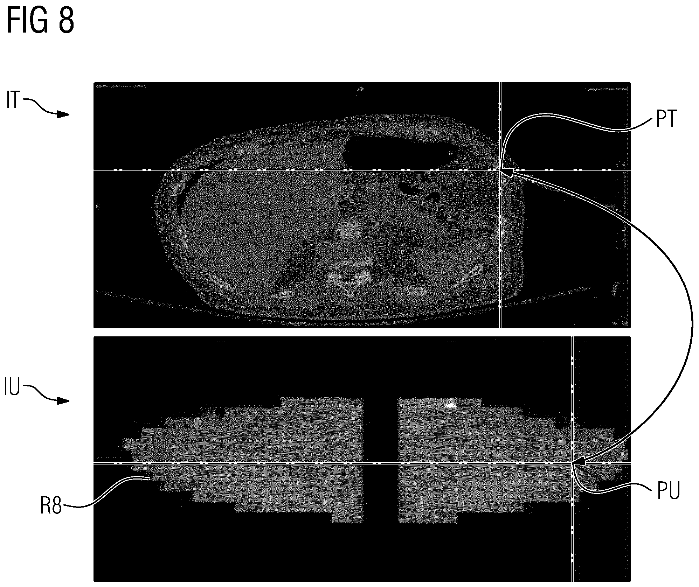

Fig. 8 eine schematische Darstellung der Zuordnung von Punkten des medizinischen 3D-Bilddatensatzes zu Punkten der zumindest einen abgerollten planaren Schicht oder des 2D-Bildes,
Fig. 9 ein 2D-Bild, welches mittels einer Ausführungsform des erfindungsgemäßen Verfahrens erzeugt wurde und welches unter Verwendung von Cinematic Rendering dargestellt wird,
Fig. 10 eine schematische Ansicht eines Ausführungsbeispiels einer erfindungsgemäßen medizinischen Bildgebungsvorrichtung.

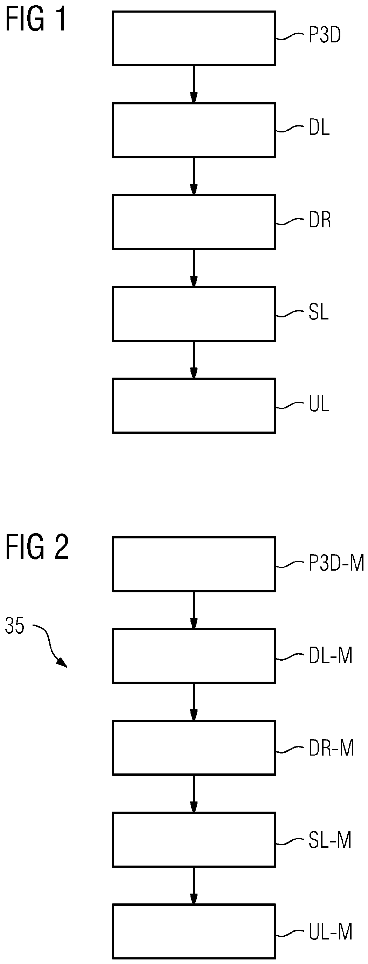

Das in Fig. 1 dargestellte Ausführungsbeispiel eines erfindungsgemäßen Verfahrens umfasst die folgenden Schritte:
- Bereitstellen P3D eines medizinischen 3D-Bilddatensatzes 3DI, welcher eine längliche anatomische Struktur RX betrifft, wobei in dem medizinischen 3D-Bilddatensatz 3DI eine Mittellinie der länglichen anatomischen Struktur RX definiert ist,
- Definieren DL zumindest einer gekrümmten Schicht in dem medizinischen 3D-Bilddatensatz 3DI, wobei sich die zumindest eine gekrümmte Schicht um die Mittellinie der länglichen anatomischen Struktur RX windet,
- Definieren DR einer Mehrzahl von Strahlen, welche jeweils radial in Bezug auf die Mittellinie der länglichen anatomischen Struktur RX verlaufen und die zumindest eine gekrümmte Schicht schneiden,
- Abtasten SL zumindest eines Teils des medizinischen 3D-Bilddatensatzes 3DI in die zumindest eine gekrümmte Schicht basierend auf der Mehrzahl von Strahlen,
- Abrollen UL der zumindest einen gekrümmten Schicht, in welche der medizinische 3D-Bilddatensatz 3DI abgetastet wurde, wobei zumindest eine abgerollte planare Schicht ermittelt wird.

Das in Fig. 2 dargestellte Ausführungsbeispiel einer erfindungsgemäßen Bilddatenverarbeitungseinheit 35 weist die folgenden Komponenten auf:
- eine Bereitstellungseinheit P3D-M, welche ausgebildet ist zum Bereitstellen P3D eines medizinischen 3D-Bilddatensatzes 3DI, welcher eine längliche anatomische Struktur betrifft, wobei in dem medizinischen 3D-Bilddatensatz 3DI eine Mittellinie der länglichen anatomischen Struktur RX definiert ist,
- eine Schicht-Definitionseinheit DL-M, welche ausgebildet ist zum Definieren DL zumindest einer gekrümmten Schicht in dem medizinischen 3D-Bilddatensatz 3DI, wobei sich die zumindest eine gekrümmte Schicht um die Mittellinie der länglichen anatomischen Struktur RX windet,
- eine Strahlen-Definitionseinheit DR-M, welche ausgebildet ist zum Definieren DR einer Mehrzahl von Strahlen, welche jeweils radial in Bezug auf die Mittellinie der länglichen anatomischen Struktur RX verlaufen und die zumindest eine gekrümmte Schicht schneiden,
- eine Abtasteinheit SL-M, welche ausgebildet ist zum Abtasten SL des medizinischen 3D-Bilddatensatzes 3DI in die zumindest eine gekrümmte Schicht basierend auf der Mehrzahl von Strahlen,
- eine Abrolleinheit UL-M, welche ausgebildet ist zum Abrollen UL der zumindest einen gekrümmten Schicht, in welche der medizinische 3D-Bilddatensatz 3DI abgetastet wurde, wobei zumindest eine abgerollte planare Schicht ermittelt wird.

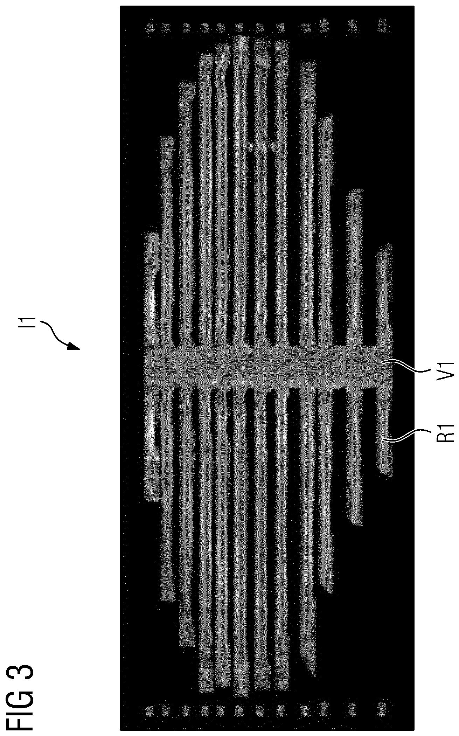

Bei der länglichen anatomischen Struktur RX kann es sich insbesondere um eine entfaltete Rippe handeln. Fig. 3 zeigt ein medizinisches Bild I1 mit entfalteten Rippen R1. Typischerweise liegen 24 entfaltete Rippen auf einem Bild vor. Es ist jeweils ein Schnittbild der Rippe mit einer Schnittebene dargestellt. Diese Schnittebene kann beispielsweise mit Hilfe eines Computer-Mausrades gedreht werden. Für eine vollständige Befundung müssen daher in der Regel ca. 90 Bilder mit jeweils 24 Rippen beurteilt werden.

Ausgehend von den extrahierten Mittellinien der Rippen R1 und/oder der Geometrie der dazugehörigen Wirbelkörper V1 können die einzelnen Rippen R1 oder Wirbelkörper V1 beispielsweise über eine Region-Growing-basierte Methode segmentiert werden. Alternativ können die Knochen auch über eine Oval-Methode segmentiert werden, bei welcher Strahlen von außen in Richtung Mittellinie CL verlaufen und beim Kontakt mit dem Knochen stoppen. Aufgrund des Grauwertverlaufs kann die Rippe in zwei Bereiche eingeteilt werden. Zum einen in den inneren Bereich AS und zum anderen in einen äußeren Bereich AC. Insbesondere kann der innere Bereich AS anatomisch der Substantia spongiosa entsprechen und der äußere Bereich AC anatomisch der Substantia corticalis entsprechen. Die Kontur ES markiert den Übergang zwischen dem inneren Bereich AS und dem äußeren Bereich AC.

Das Abtasten SL kann insbesondere über ein adaptives Raytracing erfolgen. Das Definieren DR der Mehrzahl von Strahlen PR bildet dabei ein optionales Hilfsmittel, um den zumindest einen Teil des 3D-Bilddatensatzes 3DI in die zumindest eine gekrümmte Schicht abzutasten. Das Abtasten SL kann auch unter Verwendung anderer Ansätze erfolgen, insbesondere ohne vorher eine Mehrzahl von Strahlen PR zu definieren DR.

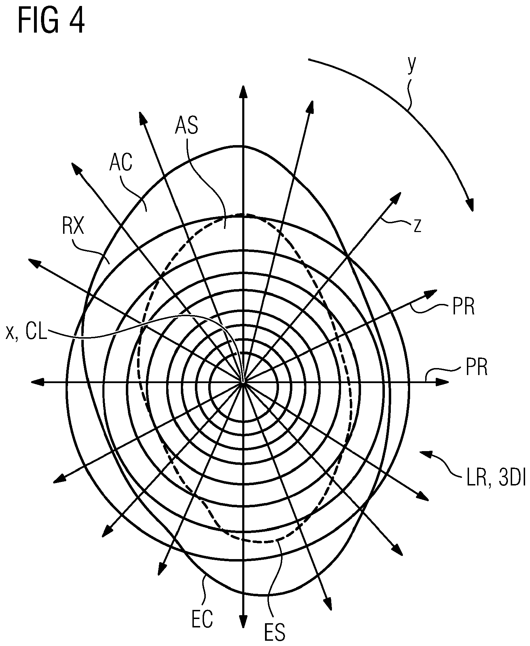

Beim radialen Raytracing, welches in der Fig. 4 dargestellt und nicht Teil der Erfindung ist, werden Samples radial bei gleichbleibender Distanz zum Zentrum, das sich auf der Mittellinie CL befindet, in die jeweilige gekrümmte Schicht LR abgetastet. Das resultierende Grauwertbild kann durch Abtastung des Originalbildes entlang der Oberfläche der Rippen in die zumindest eine gekrümmte Schicht, welche somit eine Projektionsfläche bildet, berechnet werden. Dabei wird die Mittellinie CL der Rippe auf die X-Achse abgetragen. Die zwei wählbaren Parameter sind dabei die Anzahl abgetasteter Winkel und die Abtastrate. Diese können eindeutig durch eine Projektionsvorschrift auf die Y-Achse und Z-Achse eines dreidimensionalen Volumens abgebildet werden.

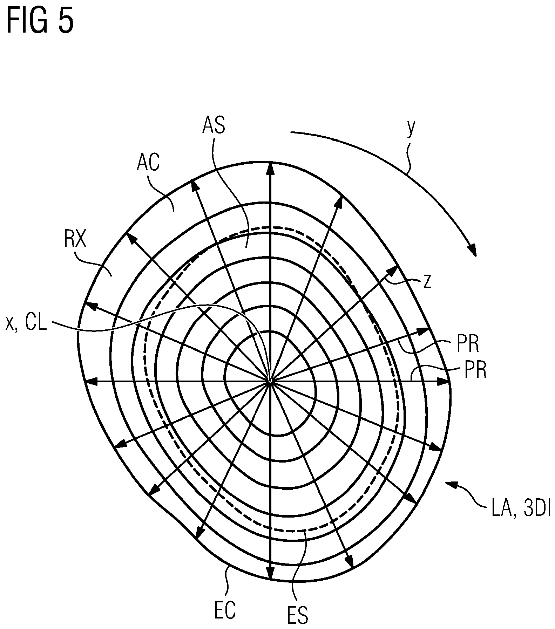

Die adaptive Raytracing-Methode ist in der Fig. 5 dargestellt. Hierzu wird die Sampling-Distanz entlang des Strahls an die Dicke der Rippe angepasst, was die Abtastung bzw. die gekrümmten Schichten LA an die Oberflächenstruktur der Rippe angleicht. Die Oberfläche EC der Rippe wird durch eine schwellwertbasierte Methode (HU) ermittelt. Sobald eine gewisse HU-Schwelle erreicht ist, beginnt die Abtastung/Übertragung der Dichtewerte indes auf gefaltete Bild. Zur Verbesserung und zur Absicherung gegen Rauschen können auch 2 oder mehrere Pixel in einer bestimmten Reihenfolge zur Ermittlung der Oberfläche herangezogen werden.

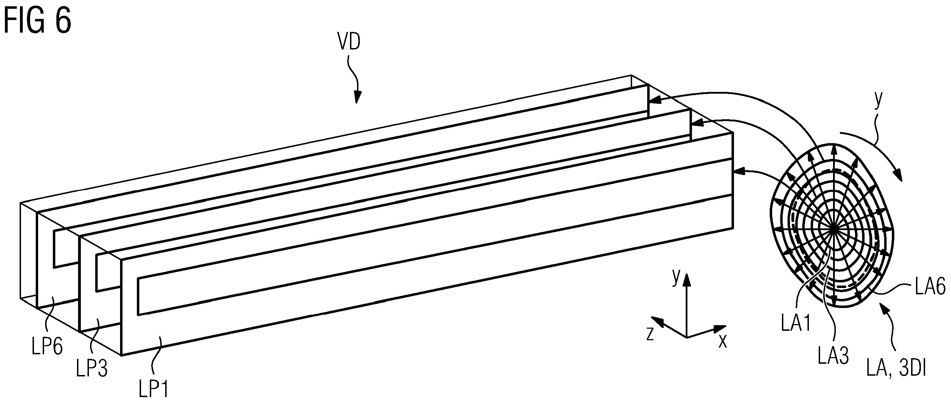

Beim Abrollen wird, wie in der Fig. 6 gezeigt, im jede gekrümmte Schicht LA1, LA3, LA6 in eine planare Schicht LP1, LP3, LP6 überführt. Von innen nach außen betrachtet, befindet sich im Zentrum die erste Schicht LA1 und an der Oberfläche der länglichen anatomischen Struktur RX die letzte Schicht, welche entlang der Z-Achse in dem Volumendatensatz VD aneinandergereiht sind. Jeder abgetastete Winkel wird entsprechend auf die Y-Koordinate abgetragen, wobei die X-Achse die Mittellinie der jeweiligen länglichen anatomischen Struktur RX beschreibt.

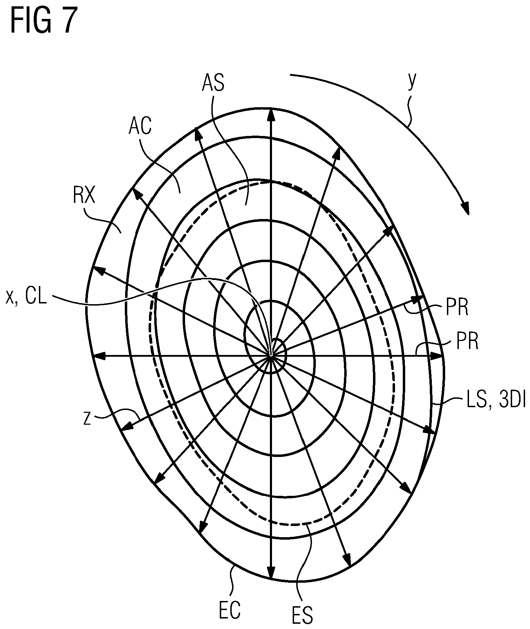

Hiermit wird ferner, außerhalb der Erfindung, ein adaptives spiralförmiges Raycasting-Verfahren offenbart, welches in der Fig. 7 dargestellt ist. Hierzu wird die Sampling-Distanz entsprechend angepasst. Die Abtastung erfolgt in einer Spiralform in die gekrümmte Schicht LS, welche sich spiralförmig um die Mittellinie CL der länglichen anatomischen Struktur RX windet. Das hat den Vorteil, dass kein Volumendatensatz als Zwischenschritt mehr erzeugt wird, sondern direkt eine Dimensionsreduzierung auf ein 2D-Bild generiert wird. Die X-Achse beschreibt dabei die Mittelinie des Knochens und die Y-Achse wird durch die spiralförmige Sampling-Abfolge bestimmt.

Für die 2D-Darstellung von mehreren benachbarten Rippen mit Hilfe der jeweiligen abgerollten planaren Schichten, welche jeweils basierend auf der spiralförmigen Schicht ermittelt wurden, ist ein Portrait-Monitor besonders vorteilhaft, da diese abgerollten planaren Schichten eine relativ große Erstreckung in die Richtung der Y-Achse aufweisen.

Basierend auf dem Volumendatensatz, wird ein 2D-Bild durch Minimum-, Maximum-Intensitäts-Projektion oder, außerhalb der Erfindung, durch Mittelung, erzeugt. Anhand der zuvor ermittelten Segmentierung der jeweiligen harten und weichen Knochenbereiche kann die Projektion für beide Segmente jeweils getrennt oder gemeinsam berechnet werden. Diese Trennung ermöglicht es, 2D-Darstellungen der beiden Bereiche zu erzeugen, indem unterschiedliche Filter der Minimum-, Maximum-Intensitäts-Projektion oder, außerhalb der Erfindung, der Mittelung auf den Volumendatensatz VD, angewandt werden.

Knochenfrakturen werden in der Minimum-Intensitäts-Projektion (MinIP) des kortikalen Knochens dadurch leichter sichtbar, während z.B. osteoplastische Knochenläsionen in der Maximum-Intensitäts-Projektion (MIP) des spongiösen Knochens sichtbar werden.

Die 2D-Darstellung erlaubt eine schnelle Orientierung und Detektion von Auffälligkeiten. Zur detaillierten Befundung in den original 3D-Bilddaten wird eine synchronisierte Rückberechnung eines markierten Pixels PU im Resultatbild IU der abgerollten Rippe R8 zur Original-Datensatzposition PT durchgeführt. Diese Position PT wird in den Multi Planar Reformation (MPR) Ansichten IT durch Referenzlinien markiert. Die synchronisierte Rückberechnung in die original 3D-Bilddaten erlaubt eine effiziente Navigation in die Originaldaten zur Detail-Befundung.

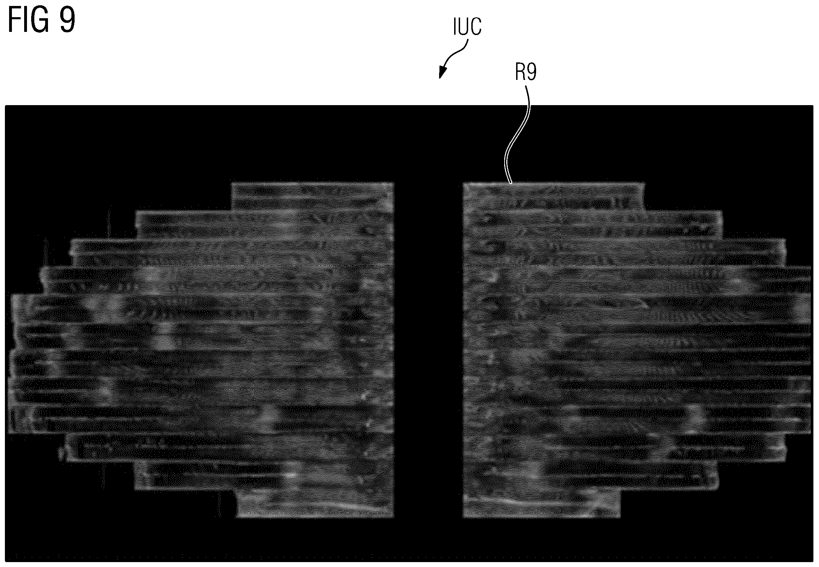

Ferner kann Cinematic Rendering zur Darstellung des 2D-Bildes, welches basierend auf der zumindest einen abgerollten planaren Schicht erzeugt wurde, verwendet werden. Mittels Cinematic Rendering kann zusätzlich zu den Filtern ein Beleuchtungsmodell zur Verfügung gestellt werden. In der Fig. 9 ist ein Beispiel zur Verwendung von Cinematic Rendering bei einem 2D-Bild IUC, welches mit Hilfe des erfindungsgemäßen Verfahrens erzeugt wurde, dargestellt. Durch die Beleuchtung der Bilder, beispielsweise von hinten, können Auffälligkeiten in den Bildern, insbesondere Frakturen der Rippen R9, deutlicher hervorgehoben werden.

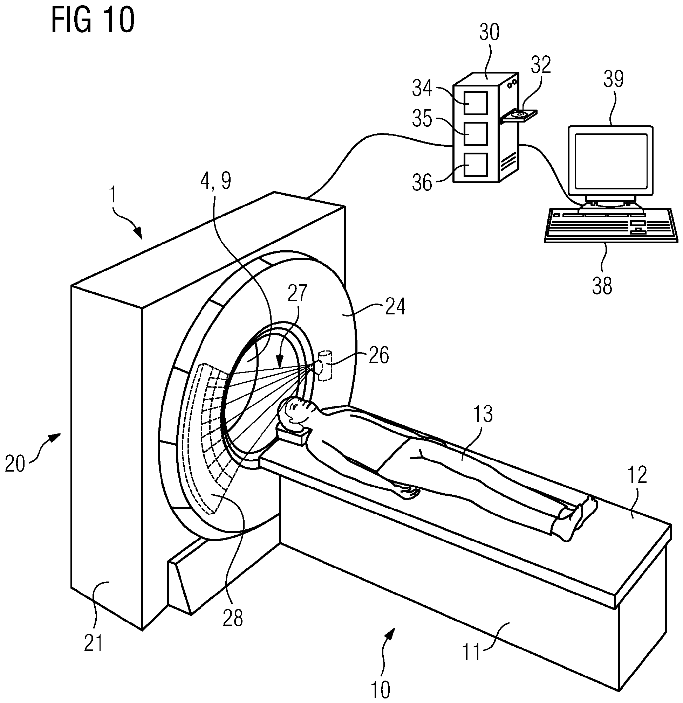

Fig. 10 zeigt eine schematische Ansicht eines Ausführungsbeispiels einer erfindungsgemäßen medizinischen Bildgebungsvorrichtung 1. Ohne Beschränkung des allgemeinen Erfindungsgedankens ist für die medizinische Bildgebungsvorrichtung 1 beispielhaft ein Computertomographiegerät gezeigt. Die medizinische Bildgebungsvorrichtung 1 weist die Gantry 20, die tunnelförmige Öffnung 9, die Patientenlagerungsvorrichtung 10 und die Steuerungsvorrichtung 30 auf.

Die Gantry 20 weist den stationären Tragrahmen 21 und den Rotor 24 auf. Der Rotor 24 ist mittels einer Drehlagerungsvorrichtung an dem stationären Tragrahmen 21 um eine Rotationsachse relativ zu dem stationären Tragrahmen 21 drehbar angeordnet. In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.

Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12, insbesondere entlang der Systemachse AR, in den Akquisitionsbereich 4 einführbar ist.

Die medizinische Bildgebungsvorrichtung 1 ist zur Akquisition von Akquisitionsdaten basierend auf einer elektromagnetischen Strahlung 27 ausgebildet. Die medizinische Bildgebungsvorrichtung 1 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor.

Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern der medizinischen Bildgebungsvorrichtung 1 ausgebildet. Die Steuerungsvorrichtung 30 weist die Bilddatenverarbeitungseinheit 35, das computerlesbare Medium 32 und das Prozessorsystem 36 auf. Die Steuerungsvorrichtung 30, insbesondere die Bilddatenverarbeitungseinheit 35, wird von einem Datenverarbeitungssystem, welches einen Computer aufweist, gebildet.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz, insbesondere 3D-Bilddatensatz 3DI, rekonstruiert werden. Die medizinische Bildgebungsvorrichtung 1 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet.

Mit der erfindungsgemäßen Lösung ist insbesondere ein Verfahren zum Rib Unfolding ("Rippen-Entfaltung") realisierbar, dass eine Dimensions-Reduktion von 3D auf 2D ermöglicht. Somit können die Rippen mittels einer relativ kompakten 2D-Darstellung visualisiert werden. Insbesondere können Knochenkompartimente, beispielsweise Kortikalis und Spongiosa, in jeweils einem separaten Volumendatensatz, welcher abgerollte planare Schichten umfasst, intuitiv dargestellt werden. Darüber hinaus wird basierend auf dem Volumendatensatz ein 2D-Bild mittels einer Projektionsmethode (Maximum-, Minimum-Intensitäts-Projektion oder, außerhalb der Erfindung, Mittelung) erzeugt.

Insbesondere können damit Spongiosa und Kortikalis separat voneinander in jeweils einem 2D-Bild vollständig dargestellt werden.

Grundsätzlich kann, außerhalb der Erfindung, dieses Verfahren auf jegliche elongierte und/oder tubuläre Strukturen angewendet verwenden. Es erlaubt insbesondere, verschiedene Layer solcher Strukturen separiert und sehr kompakt darzustellen. Die erfindungsgemäße Lösung ermöglicht insbesondere, die kortikale Begrenzung aller Rippen auf einem einzigen 2D-Bild zu visualisieren und/oder den kortikalen Knochen (hart) und den spongiosen Teil (weich) voneinander separat darzustellen. Damit kann die Befundung von beispielsweise Frakturen, Metastasen, Knochenläsionen etc. vereinfacht und beschleunigt werden.

## Patentansprüche

1. Von einem Computer ausgeführtes Verfahren zur Bilddatenverarbeitung, wobei das Verfahren die folgenden Schritte umfasst:
- Bereitstellen (P3D) eines medizinischen 3D-Bilddatensatzes (3DI), welcher eine längliche anatomische Struktur (RX) betrifft, wobei in dem medizinischen 3D-Bilddatensatz (3DI) eine Mittellinie (CL) der länglichen anatomischen Struktur definiert ist, wobei die längliche anatomische Struktur (RX) eine Rippe ist,
- Definieren (DL) zumindest einer gekrümmten Schicht (LR, LA, LS) in dem medizinischen 3D-Bilddatensatz (3DI), wobei sich die zumindest eine gekrümmte Schicht (LR, LA, LS) um die Mittellinie (CL) der länglichen anatomischen Struktur (RX) windet, wobei es sich bei der zumindest einen gekrümmten Schicht (LR, LA, LS) um eine Mehrzahl von gekrümmten Schichten handelt, wobei die gekrümmten Schichten der Mehrzahl von gekrümmten Schichten im Wesentlichen koaxial um die Mittellinie (CL) angeordnet sind und die Mittellinie (CL) der länglichen anatomischen Struktur (RX) jeweils schlauchförmig ummanteln,
- Abtasten (SL) zumindest eines Teils des medizinischen 3D-Bilddatensatzes (3DI) in die zumindest eine gekrümmte Schicht (LR, LA, LS),
- Abrollen (UL) der zumindest einen gekrümmten Schicht (LR, LA, LS), in welche der zumindest eine Teil des medizinische 3D-Bilddatensatzes (3DI) abgetastet wurde, wobei zumindest eine abgerollte planare Schicht (LP1, LP3, LP6) ermittelt wird, wobei es sich bei der zumindest einen abgerollten planaren Schicht (LP1, LP3, LP6) um eine Mehrzahl von abgerollten planaren Schichten handelt, welche zusammen einen Volumendatensatz (VD) bilden,
- wobei das Verfahren **dadurch gekennzeichnet ist, dass** die gekrümmten Schichten der Mehrzahl von gekrümmten Schichten jeweils eine Fläche repräsentieren, welche durch eine zentrische Streckung auf die Oberfläche (EC) der länglichen anatomischen Struktur (RX) abgebildet werden kann, und dass basierend auf dem Volumendatensatz ein 2D-Bild (IU) mittels einer Minimum-Intensitäts-Projektion oder Maximum-Intensitäts-Projektion erzeugt wird.

2. Verfahren nach Anspruch 1, wobei eine Mehrzahl von Strahlen (PR) definiert wird, welche jeweils radial in Bezug auf die Mittellinie (CL) der länglichen anatomischen Struktur (RX) verlaufen und welche die zumindest eine gekrümmte Schicht (LR, LA, LS) schneiden.

3. Bilddatenverarbeitungseinheit (35), aufweisend:
- eine Bereitstellungseinheit (P3D-M), welche ausgebildet ist zum Bereitstellen (P3D) eines medizinischen 3D-Bilddatensatzes (3DI), welcher eine längliche anatomische Struktur (RX) betrifft, wobei in dem medizinischen 3D-Bilddatensatz (3DI) eine Mittellinie (CL) der länglichen anatomischen Struktur definiert ist, wobei die längliche anatomische Struktur eine Rippe ist,
- eine Schicht-Definitionseinheit (DL-M), welche ausgebildet ist zum Definieren (DL) zumindest einer gekrümmten Schicht (LR, LA, LS) in dem medizinischen 3D-Bilddatensatz (3DI), wobei sich die zumindest eine gekrümmte Schicht (LR, LA, LS) um die Mittellinie (CL) der länglichen anatomischen Struktur (RX) windet,
- eine Abtasteinheit (SL-M), welche ausgebildet ist zum Abtasten (SL) zumindest eines Teils des medizinischen 3D-Bilddatensatzes (3DI) in die zumindest eine gekrümmte Schicht (LR, LA, LS),
- eine Abrolleinheit (UL-M), welche ausgebildet ist zum Abrollen (UL) der zumindest einen gekrümmten Schicht (LR, LA, LS), in welche der zumindest eine Teil des medizinische 3D-Bilddatensatzes (3DI) abgetastet wurde, wobei zumindest eine abgerollte planare Schicht (LP1, LP3, LP6) ermittelt wird,
- wobei die Bilddatenverarbeitungseinheit (35) dazu eingerichtet ist, ein Verfahren nach einem der Ansprüche 1 oder 2 auszuführen.

4. Medizinische Bildgebungsvorrichtung (1), aufweisend eine Bilddatenverarbeitungseinheit (35) nach Anspruch 3.

5. Computerprogrammprodukt mit einem Computerprogramm, welches direkt in eine Speichereinrichtung einer Bilddatenverarbeitungseinheit (35) einer medizinischen Bildgebungsvorrichtung (1) ladbar ist, mit Programmabschnitten, um alle Schritte eines Verfahrens nach einem der Ansprüche 1 oder 2 auszuführen, wenn das Computerprogramm in der Bilddatenverarbeitungseinheit (35) der medizinischen Bildgebungsvorrichtung (1) ausgeführt wird.

6. Computerlesbares Medium (32), auf welchem von einem Computer (30) einlesbare und ausführbare Programmabschnitte gespeichert sind, um alle Schritte eines Verfahrens nach einem der Ansprüche 1 oder 2 auszuführen, wenn die Programmabschnitte von dem Computer (30) ausgeführt werden.

## Claims

1. Method, executed by a computer, for image data processing, wherein the method comprises the following steps:
- providing (P3D) a 3D medical image data record (3DI), which relates to an elongated anatomical structure (RX), wherein a centre line (CL) of the elongated anatomical structure is defined in the 3D medical image data record (3DI), wherein the elongated anatomical structure (RX) is a rib,
- defining (DL) at least one curved slice (LR, LA, LS) in the 3D medical image data record (3DI), wherein the at least one curved slice (LR, LA, LS) winds around the centre line (CL) of the elongated anatomical structure (RX), wherein the at least one curved slice (LR, LA, LS) is a plurality of curved slices, wherein the curved slices of the plurality of curved slices are arranged substantially coaxially around the centre line (CL) and encase the centre line (CL) of the elongated anatomical structure (RX) in a tubular manner in each case,
- scanning (SL) at least one part of the 3D medical image data record (3DI) into the at least one curved slice (LR, LA, LS),
- unrolling (UL) the at least one curved slice (LR, LA, LS), into which the at least one part of the 3D medical image data record (3DI) has been scanned, wherein at least one unrolled flat slice (LP1, LP3, LP6) is determined, wherein the at least one unrolled flat slice (LP1, LP3, LP6) is a plurality of unrolled flat slices which together form a volume data record (VD),
- wherein the method is **characterised in that** the curved slices of the plurality of curved slices each represent a surface, which can be mapped onto the surface (EC) of the elongated anatomical structure (RX) by means of a central extension and that based on the volume data record a 2D image (IU) is generated by means of a minimum intensity projection or maximum intensity projection.

2. Method according to claim 1, wherein a plurality of beams (PR) is defined, which each run radially with respect to the centre line (CL) of the elongated anatomical structure (RX) and which intersect the at least one curved slice (LR, LA, LS) .

3. Image data processing unit (35), having:
- a provisioning unit (P3D-M), which is embodied to provide (P3D) a 3D medical image data record (3DI), which relates to an elongated anatomical structure (RX), wherein a centre line (CL) of the elongated anatomical structure is defined in the 3D medical image data record (3DI), wherein the elongated anatomical structure is a rib,
- a slice definition unit (DL-M), which is embodied to define (DL) at least one curved slice (LR, LA, LS) in the 3D medical image data record (3DI), wherein the at least one curved slice (LR, LA, LS) winds around the centre line (CL) of the elongated anatomical structure (RX),
- a scanning unit (SL-M) which is embodied to scan (SL) at least one part of the 3D medical image data record (3DI) into the at least one curved slice (LR, LA, LS),
- an unrolling unit (UL-M) which is embodied to unroll (UL) the at least one curved slice (LR, LA, LS), into which the at least one part of the 3D image medical image data record (3DI) has been scanned, wherein at least one unrolled flat slice (LP1, LP3, LP6) is determined,
- wherein the image data processing unit (35) is designed to carry out a method according to one of claims 1 or 2.

4. Medical imaging apparatus (1) having an image data processing unit (35) according to claim 3.

5. Computer program product with a computer program which can be loaded directly into a storage facility of an image data processing unit (35) of a medical imaging apparatus (1), having program portions in order to execute all steps of a method according to one of claims 1 or 2 when the computer program is executed in the image data processing unit (35) of the medical imaging apparatus (1).

6. Computer-readable medium (32) on which program portions which can be read in and executed by a computer (30) are stored, in order to execute all steps of a method according to one of claims 1 or 2 when the program portions are executed by the computer (30).

## Revendications

1. Procédé exécuté par ordinateur de traitement de données d'image, dans lequel le procédé comprend les stades suivants :
- on se procure (P3D) un ensemble (3DI) de données d'image médicale en 3D, qui concerne une structure (RX) anatomique oblongue, dans lequel dans l'ensemble (3DI) de données d'image médicale en 3D, une ligne (CL) médiane de la structure anatomique oblongue est définie, dans lequel la structure (RX) anatomique oblongue est une côte,
- on définit (DL) au moins une couche (LR, LA, LS) incurvée dans l'ensemble (3DI) de données d'image médicale en 3D, dans lequel la au moins une couche (LR, LA, LS) incurvée s'enroule autour de la ligne (CL) médiane de la structure (RX) anatomique oblongue, dans lequel la au moins une couche (LR, LA, LS) incurvée est une pluralité de couches incurvées, dans lequel les couches incurvées de la pluralité de couches incurvées sont disposées sensiblement coaxialement autour de la ligne (CL) médiane et enveloppent respectivement tubulairement la ligne (CL) médiane de la structure (RX) anatomique oblongue,
- on échantillonne (SL) au moins une partie de l'ensemble (3DI) de données d'image médicale en 3D dans la au moins une couche (LR, LA, LS) incurvée,
- on déroule (UL) la au moins une couche (LR, LA, LS) incurvée, dans laquelle on a échantillonné la au moins une partie de l'ensemble (3DI) de données d'image médicale en 3D, dans lequel on détermine au moins une couche (LP1, LP3, LP6) plane déroulée, dans lequel la au moins une couche (LP1, LP3, LP6) plane déroulée est une pluralité de couches planes déroulées, qui forment ensemble un ensemble (VD) de données en volume,
- dans lequel le procédé est **caractérisé en ce que** les couches incurvées de la pluralité de couches incurvées représentent chacune une surface, qui peut être représentée par une extension centrée sur la surface (EC) de la structure (RX) anatomique oblongue, et **en ce que** sur la base de l'ensemble de données en volume, on produit une image (IU) en 2D au moyen d'une projection d'intensité minimum ou d'une projection d'intensité maximum.

2. Procédé suivant la revendication 1, dans lequel on définit une pluralité de rayons (PR), qui s'étendent chacun radialement par rapport à la ligne (CL) médiane de la structure (RX) anatomique oblongue et qui coupent la au moins une couche (LR, LA, LS) incurvée.

3. Unité (35) de traitement de données d'image comportant :
- une unité (P3D-M) de mise à disposition, qui est constituée pour mettre à disposition (P3D) un ensemble (3DI) de données d'image médicale en 3D, qui concerne une structure (RX) anatomique oblongue, dans laquelle dans l'ensemble (3DI) de données d'image médicale en 3D est définie une ligne (CL) médiane de la structure anatomique oblongue, dans laquelle la structure anatomique oblongue est une côte,
- une unité (DL-M) de définition de couche, qui est constituée pour la définition (DL) d'au moins une couche (LR, LA, LS) incurvée dans l'ensemble (3DI) de données d'image médicale en 3D, dans laquelle la au moins une couche (LR, LA, LS) incurvée s'enroule autour de la ligne (CL) médiane de la structure (RX) anatomique oblongue,
- une unité (SL-M) d'échantillonnage, qui est constituée pour l'échantillonnage (SL) d'au moins une partie de l'ensemble (3DI) de données d'image médicale en 3D, dans la au moins une couche (LR, LA, LS) incurvée,
- une unité (UL-M) de déroulement, qui est constituée pour le déroulement (UL) de la au moins une couche (LR, LA, LS) incurvée, dans laquelle la au moins une partie de l'ensemble (3DI) de données d'image médicale en 3D a été échantillonné, dans laquelle au moins une couche (LP1, LP3, LP6) plane déroulée est déterminée,
- dans laquelle l'unité (35) de traitement de données d'image est agencée pour exécuter un procédé suivant l'une des revendications 1 ou 2.

4. Dispositif (1) d'imagerie médicale, comportant une unité (35) de traitement de données d'image suivant la revendication 3.

5. Produit de programme d'ordinateur comprenant un programme d'ordinateur, qui peut être chargé directement dans un dispositif de mémoire d'une unité (35) de traitement de données d'image d'une installation (1) d'imagerie médicale, comprenant des parties de programme pour effectuer tous les stades d'un procédé suivant l'une des revendications 1 ou 2, lorsque le programme d'ordinateur est exécuté dans l'unité (35) de traitement de données d'image de l'installation (1) d'imagerie médicale.

6. Support (32) déchiffrable par ordinateur, sur lequel des parties de programme déchiffrables et exécutables par un ordinateur (30) sont mises en mémoire pour effectuer tous les stades d'un procédé suivant l'une des revendications 1 ou 2, lorsque les parties de programme sont exécutées par l'ordinateur (30) .
